Europäisches Patentamt

European Patent Office

Office européen des brevets

(19)

(11) **EP 1 388 733 A1**

(12) **EUROPEAN PATENT APPLICATION**
published in accordance with Art. 158(3) EPC

(43) Date of publication:
**11.02.2004 Bulletin 2004/07**

(51) Int Cl.⁷: **G01N 21/84**, G06T 1/00, G01N 33/483, A23L 1/172

(21) Application number: **02728068.4**

(22) Date of filing: **16.05.2002**

(86) International application number:
**PCT/JP2002/004756**

(87) International publication number:
**WO 2002/095377 (28.11.2002 Gazette 2002/48)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **18.05.2001 JP 2001149274
18.05.2001 JP 2001149275
20.02.2002 JP 2002043704**

(71) Applicant: **Endo, Yaeta, Dr.
Matsuyama-shi, Ehime 791-8016 (JP)**

(72) Inventors:
• **ENDO, Yaeta
Matsuyama-shi, Ehime 791-8016 (JP)**

• IWAHASHI, Shigeo,
c/o Mitsubishi Chemical Corp.
Kurashiki-shi, Okayama712-805 4 (JP)
• NOMURA, Kazuo, c/o Mitsubishi Chemical Corp.
Yokohama-shi, Kanagawa 227-0033 (JP)

(74) Representative:
Forstmeyer, Dietmar, Dr. rer. nat., Dipl.-Chem. et al
Boeters & Lieck,
Bereiteranger 15
81541 München (DE)

(54) **METHOD OF SCREENING GERM FOR CELL−FREE PROTEIN SYNTHESIS AND METHOD OF PRODUCING GERM EXTRACT FOR CELL−FREE PROTEIN SYNTHESIS**

(57) The present invention provides a method for selectively collecting embryos for efficiently producing an embryo extract for the cell-free protein synthesis and a method for producing an embryo extract for the cell-free protein synthesis.

The present invention can be summarized to be a method for selectively collecting embryos for the cell-free protein synthesis wherein embryos are selectively collected, based on optical information such as color information and image information, from a mixture that is obtained by applying mechanical force to plant seeds and that contains embryos and ground endosperm, and a method for producing an embryo extract wherein embryos are extracted after washing and finely grinding the embryos collected by the method.

Fig.7

**Description**

TECHNICAL FIELD

The present invention relates generally to a method for selectively collecting embryos for the cell-free protein synthesis and a method for producing an embryo extract for the cell-free protein synthesis, and more particularly, to a method for selectively collecting embryos for efficiently producing an embryo extract, for the cell-free protein synthesis, having a high synthesis efficiency, a method for preparing an embryo extract using embryos obtained by the selective collecting method, and an embryo extract obtained by the preparation method.

BACKGROUND ART

The protein synthesis reaction occurring in a cell progresses through the following steps: First of all, gene information is transcribed from a DNA having the information to an mRNA, and a ribosome translates the information of the mRNA to synthesize a protein. At present, concerning the method for *in vitro* (e.g., in a test tube) carrying out the protein synthesis occurring originally in the cell, such methods have been actively studied that ribosomes are extracted from organisms and *in vitro* cell-free protein synthesis is carried out using these (e.g., Japanese Patent Application Laid-open Pub. No. Hei6-98790, Japanese Patent Application Laid-open Pub. No. Hei6-225783, Japanese Patent Application Laid-open Pub. No. Hei7-194, Japanese Patent Application Laid-open Pub. No. Hei9-291, Japanese Patent Application Laid-open Pub. No. Hei7-147992). *Escherichia coli*, embryo, rabbit reticulocyte, and so on, have been used as raw materials for ribosomes in these methods.

The cell-free protein synthesis system has properties comparable to a living cell in the peptide synthesis reaction rate and the accuracy of the translation reaction and is, hence, a useful method that permits obtaining an objective protein without any complex purification process. Therefore, several inventions have been disclosed concerning the enhancement of the synthesis efficiency in order to more usefully apply the synthesis system to industry. In order to enhance the industrial usefulness, however, it is important to stably keep the quality of various materials used for the synthesis system high and to supply them in large amounts as well as to consider the synthesis efficiency.

The following three reasons are thought to be main ones for the decrease phenomenon in the synthesis efficiency of protein in the cell-free system so far: (1) reduction of activitie(s) of protein synthesis factor(s) accompanied with the cell fluid extraction from an organism, (2) reduction of activities of various factors or substrate concentration (s) involved in the protein synthesis during *in vitro* synthesis reaction, (3) reduction of the translation activity as a combined result of the above (1) and (2), and so on.

A. S. Spirin et al. reported that continuously supplying amino acids as materials (for proteins), ATP, and GTP *via* an ultrafilter to a cell-free protein synthesis system prepared by the conventional method (continuous cell-free system) permitted keeping the reaction for 20 h or more and achieving a protein synthesis yield twenty times that of the conventional method (A. S. Spirin et al. (1988), Science, 242, 1162-1164). Moreover, Yokoyama et al. tried the continuous cell-free synthesis using a reaction mixture including a concentrated extract of *Escherichia coli* and a dialysis membrane and succeeded in synthesizing relatively small protein molecules such as CAT and Ras in a yield as high as 3 to 5 mg per 1 ml of the reaction mixture (Kigawa et al., The 21st Meeting of Japan Society for Molecular Biology, WID 6).

These results show that the decrease in the substrate concentration of the above (2) is one of the causes of the protein synthesis efficiency reduction phenomenon in the cell-free system. In other words, it can be explained that the protein synthesis efficiency was enhanced by preventing the decrease in amino acids and an energy source (metabolic enzymes existing in the system would decrease the substrate concentration during the reaction) as well as preventing the accumulation of metabolic products such as AMP and GMP.

On the other hand, the study on the ribosome inactivation mechanism of ricin that is a cytotoxic protein included in a castor-seed revealed that anti-viral proteins produced by a plant is an RNA N-glycosidase that is identical to ricin A chain (Y. Endo et al. (1988) Biochim. Biophys. Acta, 954, 224-226). This enzyme is a ribosome-inactivating protein (RIP) that acts on a ribosome and catalyzes the hydrolysis of one N-glucoside bond present in a specific site bound by the peptide chain-elongating factor of the RNA (e.g., 23S rRNA of *Escherichia coli*, 28s rRNA of eukaryotes) binds and, a result of this reaction, the ribosome loses the peptide chain-elongating function by the dissociation of one molecule of adenine (Y, Endo et al. (1992), TIBS, 17, 266-269). A large amount of an RNA N-glycosidase named 'tritin' exists in the endosperm of wheat seeds (W. K. Roberts et al. (1989) Biochemistry, 18, 2615-2621). Moreover, it had been known that a protein that was isolated as an antibiotic substance and is called 'thionin' is distributed widely in the plant kingdom including wheat. Recently, however, it was revealed that wheat thionin is localized in the endosperm and that this protein strongly inhibits the protein synthesis by inhibiting the translation initiation reaction (J. Brummer et al. (1994) Eur. J. Biochem., 219, 425-433; P. Hughes et al. (1997) Plant Physiol., 114, 1568). In addition, the endosperm of wheat seeds contains RNase(s), DNase(s), protease(s), and so on, at high concentrations.

Although the preparation of wheat embryos for the cell-free protein synthesis has been carried out according to the method by Erikson and Blobel (A. H. Erikson and G. Blobel (1983), Methods in Enzymol., 96, 38-50), the contamination of the endosperm including protein synthesis inhibitor(s) was inevitable as long as this method is used.

Then, as a means for improving the above (1), a method for synthesizing a protein at an enhanced reaction efficiency by suppressing the activation of an inhibitory factor that is induced along with the cytoclasis and inhibits the activitie(s) of the nucleic acid synthesis and/or protein synthesis in the cell-free protein system is disclosed in Japanese Patent Application Laid-open Pub. No. Hei7-203984. Although a means to remove the inhibitory factor from the protein synthesis system and a means for inhibiting the activation of the inhibitory factor in a protein synthesis system are exemplified as means for suppressing the activation of it in the bulletin, it is generally difficult to selectively remove inhibitory factor (s) from a protein synthesis system, so that a means for using a specific inhibitor is recommended. Concretely, an inhibitor called 'tritin' present in the wheat embryo is suppressed by a tritin antibody.

Moreover, removal of an endogenous specific inhibitor that is localized in the endosperm of wheat seeds and inhibits the protein synthesis reaction as described above by mechanical and chemical treatments is described in Japanese Patent Application Laid-open Pub. No. 2000-236896, which describes the ultrasonic treatment in water to obtain endosperm-free embryos.

A method for obtaining a cell-free extract for the cell-free protein synthesis from plant seeds is normally carried out by grinding plant seeds, removing testas and an endosperm fraction by a sieve, heavy liquid sorting, visual observation, and so on, obtaining a crude embryo fraction, removing endosperm components from the crude fraction by the washing , followed by grinding them and preparing the extract.

However, the ground plant seed consists of embryos, finely ground testas and endosperms, so that it is difficult and time-consuming to sort only embryos by the visual observation or the like, i.e., it is difficult to obtain a large amount of embryos in a short time.

DISCLOSURE OF THE INVENTION

The object of the present invention is to provide a method for obtaining embryos from plant seeds in a large amount in a short time in order to industrially and efficiently produce a cell extract for the cell-free protein synthesis system.

The inventors carried out an exhaustive research in order to achieve the above subject and found that selectively collecting embryos based on the optical information of embryo (e.g., using a color sorter or image sorter) permits efficiently obtaining highly pure embryos and efficiently producing an embryo extract for the cell-free protein synthesis using this embryo fraction. The present invention was achieved based on these findings.

In other words, the present invention provides (1) a method, for selectively collecting embryos for the cell-free protein synthesis, characterized by, based on the optical information of the embryo, selectively collecting embryos from a mixture including embryos and ground endosperm obtained by applying mechanical force to plants seeds.

A preferred embodiment of the present invention provides (2) a method described in the above (1) wherein the optical information is color information, (3) a method described in the above (1) wherein selectively collecting embryos based on the optical information is carried out by a color sorter, and (4) a method described in the above (1) wherein the color sorter is apparatus equipped with at least a means for irradiating a crude embryo fraction with light, a means for detecting the reflected light and/or transmitted light from the crude embryo fraction, a means for comparing a measurement with a reference, and a means for selectively collecting or removing ones inside or outside a range of reference.

Moreover, another embodiment of the present invention provides (5) a method described in the above (1) wherein the optical information is image information, (6) a method described in the above (1) wherein selectively collecting an embryo based on the optical information is carried out by an image sorter, (7) a method described in the above (6) wherein the image sorter is apparatus equipped with at least a means for obtaining image data of ground plant seed, a means for detecting the difference in shape of ground plant seed based on image data obtained, and a means for selectively collecting or removing ones inside or outside a range of reference based on the difference in the shape obtained, and (8) a method described in the above (7) wherein the differences in shape are differences in area, circumference, roundness, and extension.

Another embodiment of the present invention provides (9) a method for producing an embryo extract for the cell-free protein synthesis comprising steps of grinding plant seeds, selectively collecting plant embryos, washing them, finely grinding them and preparing the extract, wherein the plant embryos are selectively collected based on the optical information of embryo.

A preferred embodiment of the present invention provides (10) a method described in the above (9) wherein the optical information is color information, (11) a method described in the above (9) wherein selectively collecting embryos based on the optical information is carried out using a color sorter, (12) a method described in the above (11) wherein the color sorter is apparatus equipped with at least a means for irradiating a crude embryo fraction with light, a means for detecting the reflected light and/or transmitted light from the crude embryo fraction, a means for comparing a measurement with a reference, and a means for selectively collecting or removing ones inside or outside a range of reference, and (13) a method described in the above (9) wherein the optical information is image information.

Moreover, another preferred embodiment of the present invention provides (14) a method described in the above (9) wherein selectively collecting embryos based on the optical information is carried out using an image sorter, (15) a method described in the above (14) wherein the image sorter is apparatus equipped with at least a means for obtaining image data of ground plant seed, a means for detecting the difference in shape of ground plant seed from data obtained, and a means for selectively collecting or removing ones inside or outside a range of reference, and (16) a method described in the above (15) wherein the differences in shape are ones in area, circumference, roundness, and extension.

Preferred embodiments of the preset invention provide (17) a method described in one selected from the group consisting of the above (1) to (16) wherein the plant seeds are seeds of wheat, barley or rice (*Oryza sativa*).

In addition, another embodiment of the present invention provides (18) an embryo extract, for the cell-free protein synthesis, produced by a method described by one selected from a group consisting of the above (9) to (16).

BRIEF DESCRIPTION OF DRAWINGS

Fig. 1 illustrates the frequency distribution of RGB brightness of the endosperms of wheat seeds (under the illumination of halogen-lamp). Y-axis denotes frequency while X-axis denotes brightness.

Fig. 2 illustrates the frequency distribution of RGB brightness of the embryos of wheat seeds (under the illumination of halogen-lamp). Y-axis denotes frequency while X-axis denotes brightness.

Fig. 3 illustrates the frequency distribution of RGB brightness of the testa of a wheat seed (under the illumination of halogen-lamp). Y-axis denotes frequency while X-axis denotes brightness.

Fig. 4 is a schematic illustration of an example of apparatus for selectively collecting embryos from a crude embryo fraction (numbers: 1, crude embryo fraction-supplying means; 2, crude embryo fraction; 3, belt; 4, light source; 5, CCD sensor; 6, sucking nozzle).

Fig. 5 is a photograph of embryos derived from wheat seeds by a CCD camera.

Fig. 6 is a photograph of ground components other than embryos by a CCD camera.

Fig. 7 is a schematic illustration of an image processor for evaluation (numbers: 7, CCD camera; 8, lens; 9, specimen; 10, slide glass; 11, lighting; 12, computor for processing image).

Fig. 8 illustrates roundness and extension of embryos and ground components other than embryos obtained in Examples 1 and 3.

DETAILED DESCRIPTION

The present invention is described in more detail below.

Any plant seeds that permit obtaining embryos can be used for the present invention although seeds of a plant selected from plants belonging to the family Poaceae such as wheat, barley, and rice (*Oryza sativa*), are usually used. Among these, wheat seed is the most suitable for the present invention.

In the present invention, an embryo fraction mainly containing intact embryos is obtained from plant seeds, wherein the "intact embryos" mean embryos having at least a budding ability and the "embryo fraction" means a fraction that mainly contains intact embryos and using this permits preparing a cell extract that can be used for the cell-free protein synthesis. Plant seeds contain only a small amount of embryo. Therefore, it is preferable to remove parts other than embryo as much as possible in order to efficiently obtain embryos from seeds. Normally, first of all, mechanical force is applied to plant seeds to give a mixture including embryos, ground endosperm, and ground testa. From the mixture, ground endosperm, ground testa, and so on, are removed to give a crude embryo fraction, wherein the "crude embryo fraction" means a mixture that includes embryos as the main component as well as ground endosperm, ground testa. Mechanical force which is strong enough to separate embryos from plant seeds or so would be appropriate.

A mixture containing embryos, ground endosperm, and ground testa is obtained normally by grinding plant seeds using a known mill.

Plant seeds can be ground normally using a known mill, preferably using a mill, which applies the impact force to one to be ground, such as pin mill and hammer mill. The degree of the grinding can be determined depending on the size of the plant seed embryos to be used. For example, in the case of wheat seeds, they are normally ground so as to have the maximal length of 4 mm or less, preferably the maximal length of 2 mm or less. Moreover, the grinding is preferably carried out by the dry method.

Then, a crude embryo fraction is obtained from the obtained ground plant seed using a known grader, for example, a sieve. For example, in the case of wheat seed, a sieve with a mesh size from 0.5 mm to 2.0 mm is normally used to obtain a crude embryo fraction, and a sieve with a mesh size from 0.7 mm to 1.4 mm is preferably used. In addition, if necessary, testa, endosperm, dirt, and so on, included in the crude embryo fraction obtained, can be removed utilizing the wind force and/or static electricity.

Moreover, a crude embryo fraction can be obtained also by utilizing the difference in specific gravity among embryo, testa and endosperm, for example, by the heavy liquid sorting. The above methods can be combined in order to obtain a crude embryo fraction including embryos as many as possible.

In the present invention, embryos are selectively collected from the crude embryo fraction obtained by the above-described method based on the optical information, such as color information and image information, of the embryo. The selective collecting of embryos based on the optical information of embryo can be carried out, for example, using a color sorter for the selective collecting based on the color information or an image sorter for the selective collecting based on the image information. This permits further purifying the embryo fraction. Although embryos can also be selectively collected from ground seed based on the optical information of embryos, the ground seed includes large amounts of components other than embryo, so that the selective collecting is not efficient, and it is preferable to sort them from a crude embryo fraction.

First of all, a method using a color sorter is described that performs the selective collecting based on the color information of embryos.

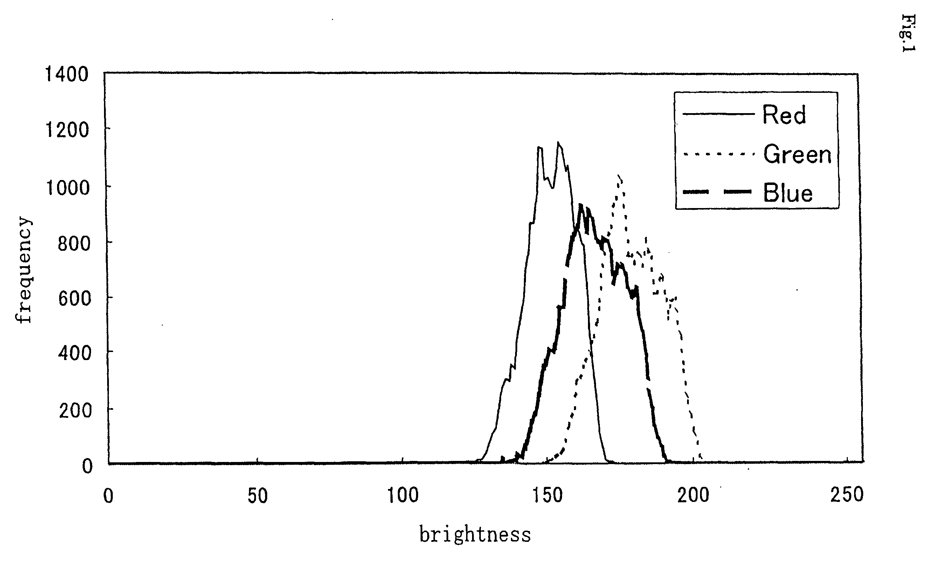

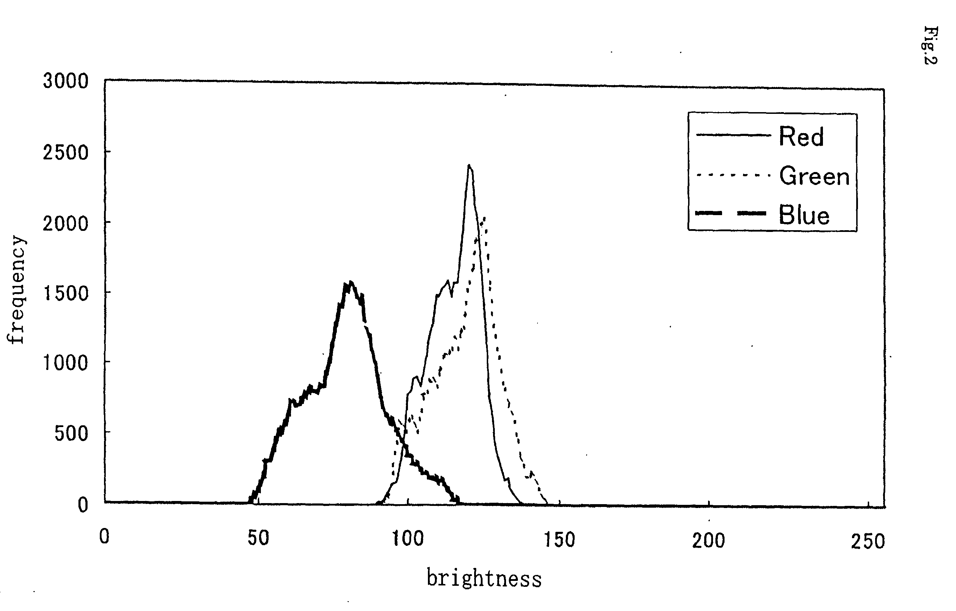

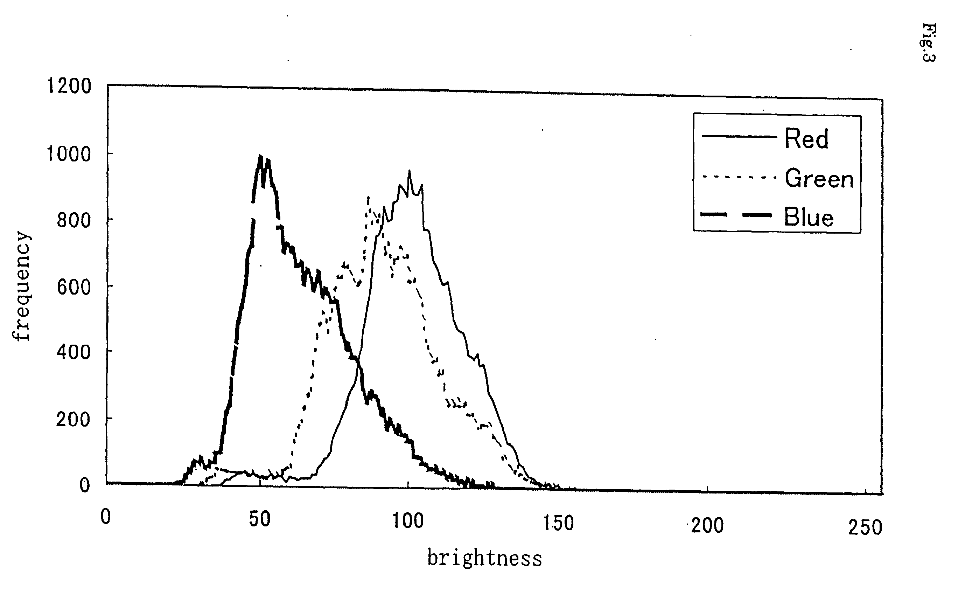

In the case of a crude embryo fraction such as a wheat seed crude embryo fraction, as illustrated in Figs. 1 to 3, for example, endosperms and embryos have different frequency distributions each other concerning RGB brightness, so that the difference permits distinguishing embryos from endosperms, wherein the RGB brightness is a method, for example, as known as an RGB color model, for expressing color information, which is adopted in a color video camera and a computor graphics system, wherein color information is expressed as brightness of R, G, and B components when the color information is decomposed into R, G, and B information, wherein R, G, and B correspond to red, green, and blue, respectively, which are three primary colors of the light. The brightness is generally expressed by 8 bits, namely by 256 steps from 0 to 255. Photographs of endosperms (Fig. 1), embryos (Fig. 2), and testas(Fig. 3) were taken by a CCD camera, and each pixel within an appropriate photographic area is decomposed into R, G, and B components, and frequency distributions concerning the brightness were determined. Embryos give a distribution of brightness that is generally smaller than endosperms particularly with respect to blue color. This means that the blue light is more absorbed by embryos. As a result, embryos look yellow. Yellow color is near the complementary color for blue color. Therefore, under the lighting of blue light, the difference in the brightness between embryo and endosperm becomes large. In this way, for example, the difference in color between embryo and endosperm is detected, for example, by the difference in the frequency distribution of RGB brightness, and embryo can be distinguished from endosperm based on it. Moreover, embryos give a higher-brightness distribution in all the components of RGB than testa, so that a difference appears in the grey scale brightness when white light lighting is used. However, the difference in the brightness of green color is the largest between embryo and testa, so that the use of lighting of green light makes the difference in brightness between them clearer. Embryos and testas give different brightness distributions against red color, so that use of yellow light that is a mixture of green light and red light can also enlarge the difference in the brightness.

Any color sorters having a function of selective collecting embryos utilizing the difference in color can be used for the present invention, wherein the sorters include apparatus equipped with at least a means for irradiating a crude embryo fraction with light, a means for detecting the reflected light and/or transmitted light from the crude embryo fraction, a means for comparing a measurement with a reference, and a means for selective collecting or removing ones inside or outside a range of reference.

Visible light or near-infrared light can be used as the light for irradiation. Incandescent lamp, fluorescent lamp, halogen lamp, or the like, can be used as the light source.

Light-intercepting sensors such as CCD sensor, silicon photosensor, germanium photosensor, and InGaAs alley sensor, can be used as a means for detecting the reflected light and/or transmitted light.

In order to practically constitute a sorter, a means for transport a crude embryo fraction also becomes necessary in addition to these functions. For the purpose, shoot-type one that permits transportation by the gravity utilizing a slope and belt conveyer can be used.

For example, a crude embryo fraction is supplied onto the belt, and the crude embryo fraction transported by the belt, launched from the end of the belt, or falling from the supplying port, can be irradiated. Kind of light source, background color (color of belt when belt is used, color of background when background is used), kind of light-receiving sensor, can be appropriately selected depending on the kind of seed to be collected or ones to be removed (embryos or others). In case of removing testas, for example, a background with similar brightness and color to the embryo (e. g., beige) and a light source of green color-based or yellow color-based light (e.g., incandescent lamp color) are preferably used. Moreover, in order to remove endosperms, a background color darker than embryo and blue color-based light source are preferably used.

Based on the reflected and/or transmitted light from the detected crude embryo fraction, by a means for comparing the measurement and the reference, it is judged whether the object is to be collected or removed. Based on the judgment, a crude embryo fraction can be collected or removed, and an embryo fraction from which parts other than embryo are removed can be obtained. For a means to compare a measurement and a reference each other, well-known means can be used.

For a means for selective collecting or removing ones inside or outside a range of reference, for example, a means for selective collecting or removing ones by sucking or jetting a fluid such as air can be used.

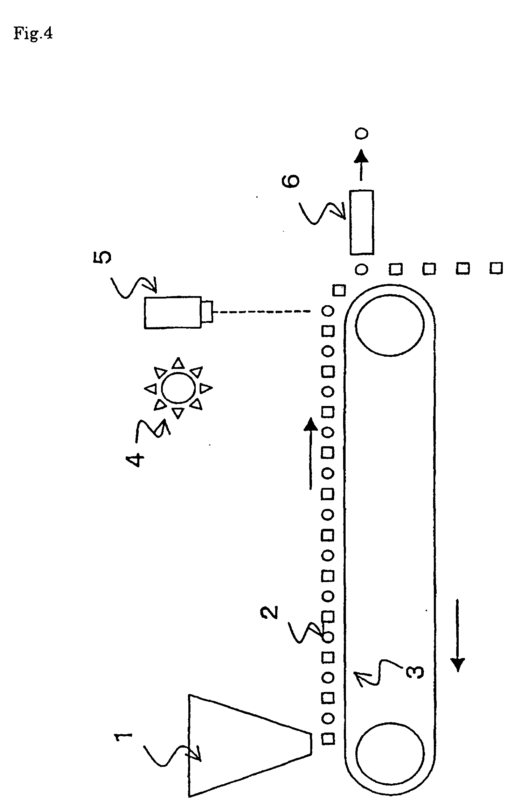

Fig. 4 is a schematic illustration of one example of apparatus for selective collecting embryos from a crude embryo fraction (numbers: 1, crude embryo fraction-supplying means; 2, crude embryo fraction; 3, belt; 4, light source; 5, CCD sensor; 6, sucking nozzle). For example, irradiating crude embryo fraction 2 supplied from crude embryo-supplying means 1 onto belt 3 and transported on belt 3, detecting reflected light by CCD sensor 5, comparing the measurement with a reference, and suck-removing ones outside a range of reference with sucking nozzle 6, permit selectively collecting embryos.

Wheat embryos have a length of about 2 mm, a width of about 1 mm, a thickness of about 0.5 mm, and a weight of about 0.5 to 0.6 mg or so, i.e., very small and light. Therefore, in order to enhance the selective collecting efficiency, it is preferable to supply a crude embryo fraction onto a belt, to irradiate the crude embryo fraction transported at a velocity of normally 5 to 100 m/min, preferably 10 to 90 m/min, and to sort them. There is no limitation to the amount of the crude embryo fraction supplied onto the belt, although the lower limit of supply amount is normally 10 particles/m$^2$, preferably 1,000 particles/m$^2$ and the upper limit of supply amount is normally 10,000 particles/m$^2$, preferably 7,000 particles/m$^2$, more preferably 5,000 particles/m$^2$ or so. An appropriate range of supply amount is normally 10 to 10,000 particles/m$^2$, preferably 1,000 to 7,000 particles/m$^2$, more preferably 1,000 to 5,000 particles/m$^2$ or so. Such limitation enhances the selective collecting accuracy.

Carrying out suck-removing in a time as short as possible and instantly permits avoiding mis-sucking embryos around matters to be removed to enhance the selective collecting accuracy. Sucking power for this operation can be generated by supplying air pressure to an ejector, for example, using an electromagnetic valve. It is preferable that the operation time of the ejector, i.e. the time when the electromagnetic valve is open, is as short as possible. The open-signal to the electromagnetic valve is normally 0.5 to 10 ms, preferably 0.5 to 2 ms. Moreover, it is also necessary to use an electromagnetic valve that responds quickly.

As means of collecting embryos, for example, components such as testas, having a thicker color than embryos are removed, followed by removing components such as endosperm, having a thinner color than embryo. These steps can be reversed. These steps can be repeated by several times. Moreover, only embryos can be collected.

Then, a method for selectively collecting with using an image sorter based on the image information of embryos is described below.

Embryo is harder than other parts, so that embryos are not ground and are isolated keeping their original shapes while other parts are ground in the above grinding treatment.

In the present invention, embryos are selectively collected from a crude embryo fraction including embryos, ground endosperm, ground testa, and so on, which was obtained as described above based on the difference in shape using an image sorter. It is also possible to sort embryos from ground seed. However, ground seeds include large amounts of components other than embryos, so that the selective collecting is not efficient. It is preferable to sort from a crude embryo fraction.

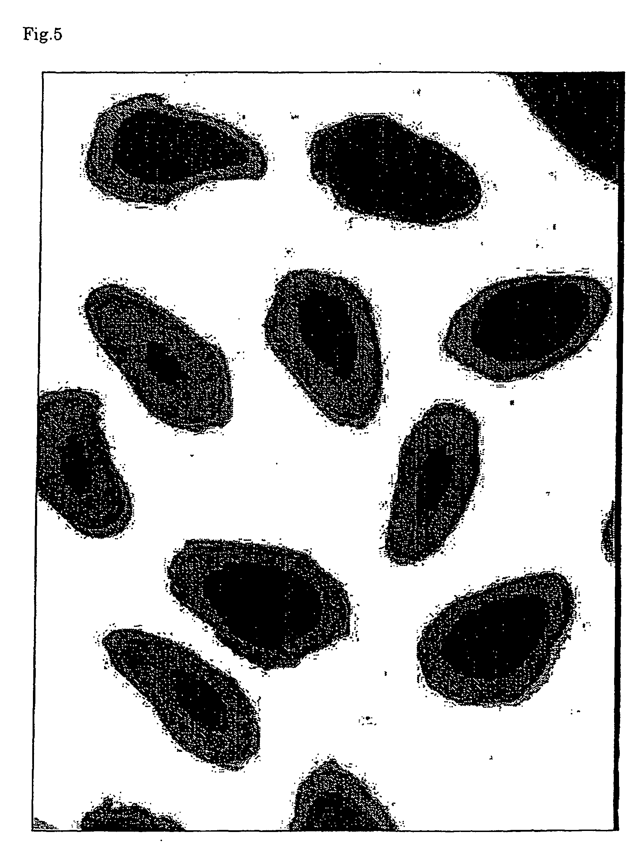

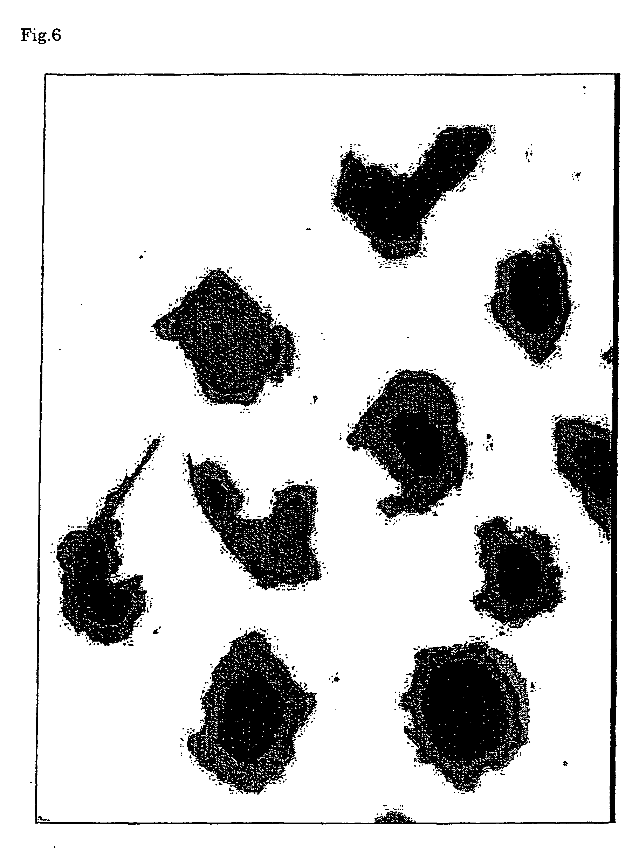

With respect to a crude embryo fraction, for example, in the case of wheat seed, as illustrated in Figs. 5 and 6, embryos and ground components other than embryos have different shapes each other. Therefore, utilizing optical information, i.e. image information, obtained from the difference permits distinguishing embryo from endosperm.

Fig. 5 is a photograph of embryos, and Fig. 6 is a photograph of ground components other than embryos by a CCD camera. Embryo has a near-oval shape, compared with ground components other than embryos. Therefore, the difference in optical information based on the difference in shape can be judged as the difference, for example, in area, circumference, roundness, extension, and so on, and embryos can be selectively collected from ground components other than embryos based on the difference.

Any image sorter can be used for the present invention as long as it has a function of selectively collecting embryos utilizing the image information based on the difference in shape. For example, such apparatus can be used which has at least a means for obtaining the image data of ground plant seed, a means for recognizing shapes of plant ground seed from the image data obtained, and a means for selectively collecting or removing ones inside or onside a range of reference based on the difference in shape obtained.

Concretely, for example, a crude embryo fraction supplied onto and transported on a belt is irradiated with visible or near-infrared light using incandescent lamp, fluorescent lamp, halogen lamp, or the like, and image data are obtained from reflected and/or transmitted light from the crude embryo fraction using a light-intercepting sensor such as CCD sensor and CMOS sensor. Kind of light source, background color (e.g., color of belt when belt is used, color of background when background is used), kind of light-intercepting sensor, and so on, can be selected in a combination that permits easily detecting the difference in shape depending on kind of seed to be collected and what is to be removed (i.e., embryo or other than embryos).

Difference in shape of ground plant seed is determined from the image data obtained. A measurement representing the shape is determined by the image processing of the image data obtained, and the measurement is compared with a predetermined reference to determine the difference in shape. Measurements representing the shape include area, circumference, roundness, extension, and so on. For means for the determination, well-known ones can be used.

For example, using an appropriate computer system, the binary coded processing and the noise removal are carried out to the image data obtained, and a roundness value and an extension value are calculated by an analyzing means such as blob analyzing. A roundness value and an extension value by blob analyzing can be determined as follows:

$$\text{Roundness value} = \frac{\text{blob circumference}}{2\sqrt{\Pi} \times \text{blob area}} \tag{1}$$

$$\text{Extension value} = \frac{(\text{blob longest axis})^2}{\text{blob area}} \tag{2}$$

The above image processing (blob analyzing) can be carried out using commercially available softwares such as measurement/control software "LabVIEW ver.6.0" (National Instruments Corp.) and add-on image processing tool "MAQ Vision ver.6.0.1" for LabVIEW. The blob analyzing is described in detail in Chapter 9 "Binary Morphology" and Chapter 10 " "Particle Measurement" in "IMAQ Vision Concept Manual" (October 2000 Edition).

The more the shape is near circle, the more the roundness value calculated in the above analyzing is near 1. The more the shape is slender, the more the extension value is large.

A roundness value and an extension value of an embryo are determined previously, and measurements are compared with these values as references. Although shape-representing measurements such as area, circumference, roundness, extension, and so on, can be used singly, using these in a combination, for example, by the multivariate analysis permits enhancing the selective collecting accuracy.

The multivariate analysis can be applied, for example, according to the following procedure: beforehand, blob analysis is carried out for embryos and a fraction, which is to be removed, other than embryos, and then the following regression equation (3) is determined using 'm' kinds of measurements obtained. The measurements include the above area, circumference, roundness, and extension; blob longest axis, equal area elliptical long axis, equal area elliptical short axis, hydraulic radius, value(s) obtained by the arithmetic operation of these (e.g., equal area elliptical long axis/ equal area elliptical short axis), and so on.

$$Y = A0 + A1 \times \textit{Measurement } 1 + A2 \times \textit{Measurement } 2 +$$

$$\cdots + Am \times \textit{Measurement } m \tag{3}$$

Regression is carried out, with $Y$ being 1 in case the specimen for blob analysis is an embryo in regression formula (3), with $Y$ being 0 in case the specimen is a part other than embryo, to determine constants $A1$ to $Am$ that are weights for each measurement. For the regression, although a method that is known as linear multiple regression can be used, if the partial least square (PLS) method is used, selective collecting accuracy is more enhanced.

After $A0$ to $Am$ are determined, blob analysis is carried out for an unknown specimen. In case $Y$ is determined from the above regression formula (3), if the specimen is an embryo, $Y$ becomes a value near 1. If the specimen is not an embryo, $Y$ becomes a value near 0. Thus, selective collecting becomes possible.

An embryo fraction from which ingredients other than embryos are removed is obtained by selectively collecting or removing ones inside or outside a range of reference based on the difference in shape obtained by the above methods or the like. For a means for selectively collecting or removing ones outside or inside a range of reference, for example, a means for selectively collecting or removing them by sucking or ejection fluid such as air can be used.

Apparatus equipped with the above means can be apparatus similar to one provided in Fig. 4 as an example of apparatus for selectively collecting embryos from a crude embryo fraction (numbers: 1, crude embryo fraction-supplying fraction; 2, crude embryo fraction; 3, belt; 4, light source; 5, CCD sensor; 6, sucking nozzle). Embryos can be collected, for example, by irradiating crude embryo fraction 2 that is supplied from crude embryo fraction-supplying means 1 onto belt 3 and transported by belt 3, obtaining image data using CCD sensor 5, comparing a measurement (obtained by image data )with a range of reference, followed by suck-removing ones outside the reference's range by sucking nozzle 6.

As described above, wheat embryo has a length of about 2 mm, a width of about 1 mm, and a thickness of about 0.5 mm, and a weight of about 0.5 to 0.6 mg, i.e., very small and light. Therefore, in order to enhance the selective collecting accuracy, it is preferable to carry out the selective collecting by supplying a crude embryo fraction onto a belt, followed by irradiating the crude embryo fraction transported by the belt at normally 5 to 100 m/min, preferably 10 to 90 m/min. There is no limitation to the amount of the crude embryo fraction supplied onto the belt, although the lower limit of the amount supplied is normally 10 particles/m$^2$, preferably 1,000 particles/m$^2$, and the upper limit is 10,000 particles/m$^2$, preferably 7,000 particles/m$^2$, more preferably 5,000 particles/m$^2$. The selective collecting accuracy can be enhanced by using such conditions.

This procedure can be repeated several times if necessary. Moreover, only embryos can be collected.

As described above, carrying out the suck-removal in a time as short as possible and instantly permits avoiding mis-sucking embryos near matters to be removed and enhancing the selective collecting accuracy. The sucking power for the operation can be generated by supplying air pressure to en ejector, for example, using an electromagnetic valve. It is preferable that the time when the ejector is operating, i.e. the time when the electromagnetic valve is open, is as short as possible. It is preferable that the open signal to the electromagnetic valve is 0.5 to 10 ms, preferably 0.5 to 2 ms. Moreover, it is also necessary to use an electromagnetic valve that responds quickly.

As described above, it is possible to efficiently sort-purify embryos in a large amount based on the optical information of the embryo.

An endosperm component is sometimes attached to thus obtained embryo fraction. Therefore, it is preferable to further wash the product to purify the embryo.

For the washing treatment, it is preferable to disperse or suspend the embryo fraction in water or water solution cooled normally down to 10°C or lower, preferably 4°C or lower and to wash it until the washing solution does not become cloudy. It is more preferable to disperse or suspend the embryo fraction in a surfactant solution cooled normally down to 10°C or lower, preferably 4°C or lower and to wash it until the washing solution does not become cloudy. Surfactants used are preferably non-ionic ones such as polyoxyethylene derivatives such as Brij, Triton, Nonidet P40, and Tween. Among these, Nonidet P40 is the best. These non-ionic surfactants can be used, for example, as a 0.5% solution or so.

Both the washing treatment with water or water solution and the washing treatment with a surfactant can be carried out, and one of these treatments can also be carried out. Moreover, these washing treatments can be carried out in combination with the ultrasonic treatment.

Thus obtained embryos are washed, finely ground and treated to give an embryo extract. The embryo extraction can be carried out by one of the known methods. For example, embryos frozen with liquid nitrogen were finely ground and mixed with an extract solution, and the embryo mixture is centrifuged to obtain embryo extract, and then the obtained extract can be purified by the gel filtration or the like if necessary.

A solution containing buffer(s), potassium ion, magnesium ion and/or antioxidant having thiol group(s) can be used as the extracting solvent. Calcium ion, L-amino acid(s), and so on, can be further added to the solution if necessary. For example, a solution containing N-2-hydroxyethylpiperazine-N'-2-ethanesulfonic acid (HEPES)-KOH, potassium acetate, magnesium acetate, L-amino acid(s) and/or dithiothreitol or a solution obtained by modifying a procedure according to Patterson et al. (HEPES-KOH, potassium acetate, magnesium acetate, calcium chloride, L-amino acid(s) and/or dithiothreitol) can be used as the extract solution. The composition/concentration of each component in the extract solution is known, and a solvent normally used for preparing an embryo extract for the cell-free protein synthesis can be adopted.

The gel filtration can be carried out using gel filtration column that had been equilibrated with equilibrium solution (i.e., containing HEPES-KOH, potassium acetate, magnesium acetate, dithiothreitol or L-amino acid(s)). The composition/concentration of each component in the solution for gel filtration is known, and one normally used for preparing the embryo extract for the cell-free protein synthesis can be adopted.

Since an embryo extract after the gel filtration can be contaminated with microorganism(s), in particular, spore (s) of fungi, it is preferable to remove these microorganisms. Since microorganism(s) can grow during the cell-free protein synthesis reaction for a long period (e.g., one day or longer), it is important to inhibit it/them. There is no limitation to the means for removing microorganism(s) although it is preferable to use a filter for sterilization. The pore size of the filter is normally 0.1 to 1 μm, preferably 0.2 to 0.5 μm. Since the spores of *Bacillus subtilis* are small, i.e., 0.5 μm x 1 μm, using a filter (e.g., Minisart™ produced by Sartorius AG) having a pore size of 0.20 μm is effective also for removing such spores. When filtration is carried out, it is preferable that pre-filtration is carried out using a filter having a larger size before main filtration using a filter capable of removing microorganisms that can be contaminants.

Thus obtained cell extracts is a purified cell extract without endosperm components, which include endogenous factors that act on ribosomes and inhibit protein synthesis, such as tritin, thionin, RNase, Dnase, protease and so on. "Cell extract without endosperm components" means an embryo extract from which endosperm was removed.

This can be judged if the ribosome is not substantially deadenylated by tritin. "To be not substantially deadenylated" means an adenylation coefficient of ribosome is less than 7%, preferably less than 1%, more preferably less than 0.1%, most preferably less than a detection limit.

Protein synthesis using a cell-free protein synthesis system can be carried out in a manner similar to the conventional method except using the embryo extract obtained as described above. This method can be a well-known batch method or a continuous amino acid- and energy source-supplying system such as the above continuous cell-free protein synthesis system by Spirin et al.

Since the protein synthesis reaction can be stopped if the reaction is carried out in a batch method for a long time, using the latter continuous amino acid- and energy source-supplying system permits carrying out the reaction for a long time and making the process more efficient.

Moreover, in case the protein synthesis is carried out in a continuously supplying system, a dialysis method can also be used. For example, in an ultrafiltration dialysis system, which use an intramembrane buffer including the embryo extract according to the present invention and an extramembrane buffer including a mixture of energy source (s) and amino acids, a large amount of proteins can be continuously produced. In the dialysis method, synthetic substrates such as energy source(s) and amino acids are supplied into the intramembrane buffer while reaction by-products and so on are removed to the extramembrane buffer (through dialysis membrane). For the dialysis membrane, known ones that are normally used can be used. Concerning the molecular weight limit of the dialysis membrane, an appropriate one can be used depending on the molecular weight of a protein to synthesize.

The energy sources include adenosine 5'-triphosphate (ATP), guanosine 5'-triphosphate (GTP), creatine phosphate, and so on. The amino acids include twenty L-amino acids.

A protein synthesized in the cell-free system can be isolated/purified from the reaction system by a known method usually used. Isolation/purification of a protein can be carried out, for example, by affinity chromatography, gel filtration chromatography, ion exchange chromatography, or the like.

Examples

Although the present invention is described in more detail with the following examples, these examples are provided as help for concretely recognizing the present invention, not to limit the scope of the present invention.

Example 1

Preparation of crude embryo fraction

90 kg of wheat seeds (Chihoku, produced in Hokkaido : not sterilization) was added to a mill (Rotor Speed Mill pulverisette Tye-14, A. FRITSCH GmbH & Co. KG) at a rate of 200 g/min, and the seeds were gently ground at 7,000 rpm. After single step of this disruption, many wheat seeds remained without separating embryos. Therefore the step was repeated four times. After a fraction containing embryos having germinability was collected with a sieve (mesh size 0.71 mm to 1.00 mm), impurities such as testa were removed by the wind power sorting using winnowing fan. Then, a floating fraction including embryos having germinability is collected by the heavy liquid sorting using a (carbon tetrachloride: cyclohexane) (2.4:1, v/v) mixture, and the organic solvent is removed by the drying at room temperature, and impurities such as testa were removed by the wind force sorting using winnowing fan to give a crude embryo fraction. After these procedures, a ratio (embryo: crude embryo fraction) became 60% or so on a weight basis.

Example 2

Extraction of color information in RGB color space

A crude embryo fraction obtained in Example 1 was photographed using a digital microscope VH-7,000 and a long distance zoom lens VH-735 (Keyence Corp.) to give a color image. Photographic conditions are as follows: halogen lamp brightness, maximum; white balance setting, 3200 K; shutter speed, 1/120 sec; lens magnification, 50-fold).

The photographic image was subjected to the color analysis by RGB color space using softwares LabVIEW ver.6.0 and IMAQ Vision ver.6.0.1 (National Instruments Corp.). First of all, images of parts that can be recognized as embryos by the visual observation were cut out, and a histogram of brightness of each component of R, G, and B was calculated using all the pixels contained in the image cut out as the population. Histograms of endosperm and testa are also calculated similarly to give results illustrated in Figs. 1 to 3.

These results showed, as described in "Embodiment of the invention", that appropriately selecting photographic conditions such as lighting permits selective collecting embryos based on the difference in the color information.

Example 3

Selectively collecting embryo using color sorter

Embryos were selectively collected from a crude embryo fraction utilizing the difference in color, as described below, using a belt-type color sorter (BLM-300K, produced by Anzai Seisakusho Co., Ltd., commercially available from Anzai Sogyo Co., Ltd.). This color sorter is apparatus similar to one illustrated in Fig. 4.

The crude embryo fraction obtained in Example 1 is supplied onto a beige belt at a density of about 5,000 particles/$m^2$ and irradiated with a fluorescent lamp to detect the reflected light. The belt was transported at a speed of 50 m/min. A monochromatic CCD line sensor (2,048 pixels) was used as a light-intercepting sensor.

First of all, in order to remove components (e.g., testa) darker than embryo, a beige belt was installed, and a fluorescent lamp giving incandescent lamp-like light was used for lighting, and a reference value is set between the brightness of embryo and the brightness of testa, and ones outside a range of reference were removed by the sucking. This step was repeated six times. Then, in order to sort endosperm, the belt was replaced with a dark-green one, and the lamp was replaced with a blue fluorescent lamp, and a reference is set between the brightness of embryo and the brightness of endosperm, and ones outside a range of reference were removed by the sucking. This step was repeated seven times. The suck-removing was carried out by sucking testa or embryo launched and falling from the end of the belt with thirty sucking nozzles (one sucking nozzle is installed per 1 cm) installed at the position of about 1cm forward of pulleys on which a transporting belt is supported. Finally, embryos that were broken and became small in the color sorting step were removed with a sieve having a mesh size of 0.71 mm.

Among the above steps, it takes 15 h for the color sorting step, 500 g of wheat embryos having a purity of 98.3%(w/w) could be obtained.

Example 4

Selectively collecting embryo based on image processing

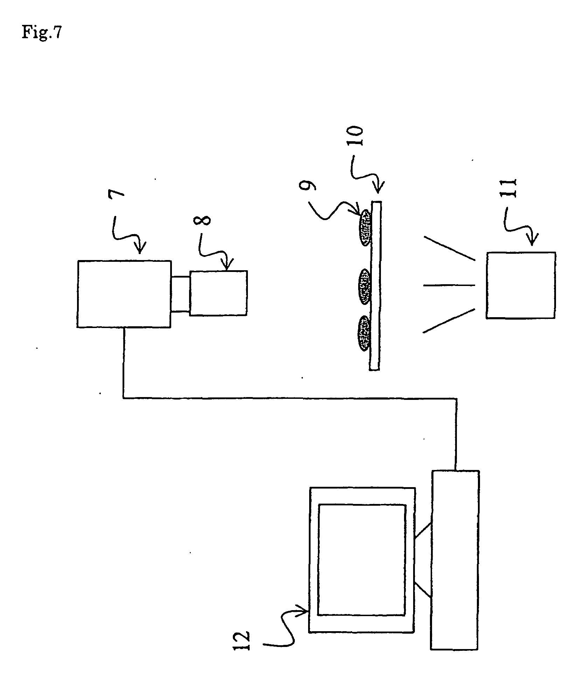

Using an image processor illustrated in Fig. 7, one-hundred twenty-three embryo specimens obtained in Example 3 and 83 specimens other than embryo in the crude embryo fraction obtained in Example 1 were evaluated with respect to the difference in shape.

Embryo specimens prepared or specimens other than embryo were mounted on a slide glass and were irradiated through the slide glass, and shadows of specimens were photographed with a 3.5 billion pixel monochromatic CCD camera. Figs. 5 and 6 illustrate examples of photographic images.

Photographic images were processed using softwares LabVIEW ver.6.0 and IMAQ Vision ver.6.0.1 (National Instruments Corp.). First of all, after the binary coded processing and the noise removal were carried out, a roundness value and an extension value were calculated by the blob analysis treatment.

The more the shape is near complete round, the more the roundness value is near 1. The more the shape is slender, the more the extension value is large.

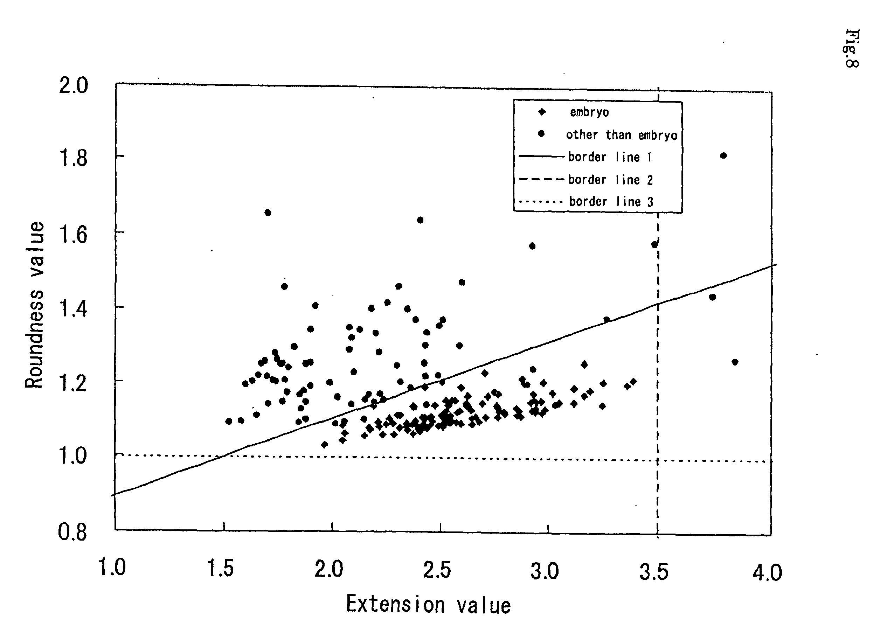

Roundness values vs. extension values of all the specimens of embryos and parts other than embryo are plotted to give Fig. 8. Embryos have similar shapes one another, so that both roundness values and extension values exist at a high density inside an area. On the other hand, specimens other than embryo give random shapes during the grinding, so that roundness values and extension values are more diverse. This difference permits selectively collecting embryos. For example, as illustrated in Fig. 8, which illustrates roundness value vs. extension value plots, inside the area surrounded by three border lines, 106 specimens of embryo exit while only 3 specimens other than embryo exist. In a word, the purity of embryo could be enhanced from about 60% (crude embryo fraction, before the image processing) to about 97% by the image processing sorting.

In case one wants to enhance the purity of the embryo, one can narrow the area surrounded by border lines. In case one wants to enhance the yield of embryo at the expense of purity, one can widen the area surrounded by border lines.

Example 5

Selective collecting of embryo by visual observation

The crude embryo fraction obtained in Example 1 was visually observed, and embryos were manually collected with a bamboo skewer to give 1 g of embryo per 60 min.

Example 6

Preparation of wheat embryo extract

The wheat embryo fraction obtained in Example 3 was suspended in distilled water kept at 4°C and washed with an ultrasonic cleaner until the washing solution does not become cloudy. Then, embryos were suspended in 0.5% (v/v) Nonidet P40 solution and washed with an ultrasonic cleaner until the washing liquid does not become cloudy to give wheat embryos.

A wheat embryo extract was prepared according to the conventional method (Erickson, A. H. et al. (1996) Meth. in Enzymol., 96, 38-50). The following procedures were carried out at 4°C. First of all, wheat embryos freezed with liquid nitrogen were finely ground in a mortar. One milliliter of an extracting solvent (80 mM HEPES-KOH (pH 7.6), 200 mM potassium acetate, 2 mM magnesium acetate, 4 mM calcium chloride, 0.6 mM each of twenty L-amino acids and 8 mM dithiothreitol) that was prepared by partially modifying the method of Patterson et al. was added to 1 g of the obtained powder, and the obtained mixture was carefully stirred not to generate a foam. Supernatant obtained by the centrifugation at 30,000 x g for 15 min was collected as an embryo extract and subjected to gel filtration using a Sephadex G-25 column that had been beforehand equilibrated with a solution (40 mM HEPES-KOH (pH 7.6), 100 mM potassium acetate, 5 mM magnesium acetate, 0.3 mM each of twenty L-amino acids and 4 mM dithiothreitol). The concentration of the specimen was prepared to give an optical density (O.D.) at 260 nm ($A_{260}$) of 170 to 250 ($A_{250}/A_{260}$ = 1.5).

Example 7

Continuous wheat embryo cell-free protein synthesis by dialysis method

Continuous embryo cell-free protein synthesis was carried out according to the method (Endo, Y. et al., (1992) J. Biotech., 25, 221-230).

The wheat embryo extract obtained in Example 6 was placed into a disposable dialyzer (Spectra/PorRCE, MWCO: 25k, volume: 0.5 ml) and dialyzed at 20°C against 10 parts volume of a dyalisis buffer (20 mM HEPES-KOH (pH 7.6), 95 mM potassium acetate, 2.65 mM magnesium acetate, 4 mM dithiothreitol, 1.2 mM ATP, 0.25 mM GTP, 16 mM creatine phosphate, 0.380 mM spermidine, 0.3 mM each of twenty L-amino acids and 1 ml/ml of 0.005% $NaN_3$) per one part volume of reaction mixture.

Synthesis of green fluorescent protein (GFP) was tried under the conditions obtained by the above methods. Fluorescence was observed at 510 nm for excitation at 490 nm, i.e., synthesis of GFP was confirmed.

INDUSTRIAL APPLICABILITY

The methods according to the present invention permit efficiently selectively collecting a large amount of embryo, and using the embryo fraction permits efficiently producing a high-quality embryo extract. The method according to the present invention is very useful for preparing a protein with a cell-free system, for example, for producing an embryo extract for the cell-free protein synthesis used for the expression of a protein in the gene function analysis, the preparation of a new enzyme or antibody in the molecular evolution technology, and so on.

CROSS-REFERENCE TO RELATED APPLICATIONS

This application claims the benefit of priority to Japanese Patent Applications (JPA2001-149274, JPA2001-149275) filed May 18, 2001, and to Japanese Patent Application (JPA2002-043704) filed February 20, 2002, of which full contents are herein incorporated by reference.

**Claims**

1. A method for selectively collecting embryos for the cell-free protein synthesis, wherein embryos are selectively collected, based on optical information of the embryos, from a mixture that is obtained by applying mechanical force to plant seeds and that contains embryos and ground endosperm.

2. The method according to claim 1, wherein the optical information is color information.

3. The method according to claim 1, wherein the selectively collecting embryos based on optical information is carried out using a color sorter.

4. The method according to claim 1, wherein the color sorter is an apparatus comprising at least means for irradiating a crude embryo fraction with light, means for detecting the reflected light and/or transmitted light from the crude embryo fraction, means for comparing a measurement with a reference, and means for selectively collecting or removing ones inside or outside a range of reference.

5. The method according to claim 1, wherein the optical information is image information.

6. The method according to claim 1, wherein the selectively collecting embryos based on optical information is carried out using an image sorter.

7. The method according to claim 6, wherein the image sorter is an apparatus comprising at least means for obtaining image data of a ground plant seed, means for detecting the difference in shape of ground plant seed from the image data obtained, and means for selectively collecting or removing ones inside or outside a range of reference, based on the difference in shape obtained.

8. The method according to claim 7, wherein the difference in shape is area, circumference, roundness, and/or extension of ground plant seed.

9. A method for producing an embryo extract for the cell-free protein synthesis, comprising the steps of grinding plant seeds, selectively collecting plant embryos, washing, fine grinding and extracting, wherein the selectively collecting plant embryos is carried out based on optical information of the embryos.

10. The method according to claim 9, wherein the optical information is color information.

11. The method according to claim 9, wherein the selectively collecting embryos based on optical information is carried out using a color sorter.

12. The method according to claim 11, wherein the color sorter is an apparatus comprising at least means for irradiating a crude embryo fraction with light, means for detecting the reflected light and/or transmitted light from the crude embryo fraction, means for comparing a measurement with a reference, and means for selectively collecting or removing ones inside or outside a range of reference.

13. The method according to claim 9, wherein the optical information is image information.

14. The method according to claim 9, wherein the selectively collecting embryos based on optical information is carried out using an image sorter.

15. The method according to claim 14, wherein the image sorter is an apparatus comprising at least means for obtaining image data of a ground plant seed, means for detecting the difference in shape of ground plant seed from the image data obtained, and means for selectively collecting or removing ones inside or outside a range of reference, based on the difference in shape obtained.

16. The method according to claim 15, wherein the difference in shape is area, circumference, roundness, and/or extension of ground plant seed.

17. The method according to any one claim selected from a group consisting of claim 1 to 16, wherein the plant seed is selected from a group consisting of wheat, barley and rice (*Oryza sativa*).

18. An embryo extract for the cell-free protein synthesis, wherein the embryo extract is produced by the method according to any one of claims 9 to 16.

Fig.1

Fig.2

Fig.3

Fig.4

Fig.5

Fig.6

Fig.7

Fig.8

## INTERNATIONAL SEARCH REPORT

| | International application No. |
|---|---|
| | PCT/JP02/04756 |

A. CLASSIFICATION OF SUBJECT MATTER
Int.Cl⁷ G01N21/84, G06T1/00, G01N33/483, A23L1/172

According to International Patent Classification (IPC) or to both national classification and IPC

B. FIELDS SEARCHED

Minimum documentation searched (classification system followed by classification symbols)
Int.Cl⁷ G01N21/84, G06T1/00, G01N33/483, A23L1/172-1/20, C12N15/09,
C12P21/00, B07B15/00

Documentation searched other than minimum documentation to the extent that such documents are included in the fields searched

| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)
JICST FILE(JOIS), search terms used: HAIGA, GAZOSHORI (in Japanese)

C. DOCUMENTS CONSIDERED TO BE RELEVANT

| Category* | Citation of document, with indication, where appropriate, of the relevant passages | Relevant to claim No. |
|---|---|---|
| Y | JP 2000-236896 A (Mitsubishi Chemical Corp., Yaeta ENDO), | 1,2,5,9,10, 13,16-18 |
| A | 05 September, 2000 (05.09.00), Full text; Figs. 1 to 7 (Family: none) | 3,4,6-8,11, 12,14,15 |
| Y | JP 63-27997 B2 (Sankai no Tomo Yugen Kaisha), | 1,2,5,17,18 |
| A | 06 June, 1988 (06.06.88), Full text; Figs. 1 to 3 (Family: none) | 3,4,6-16 |
| Y | JP 2000-202788 A (Matsushita Electric Industrial Co., Ltd.), | 1,2,5,9,10, 13,16-18 |
| A | 25 July, 2000 (25.07.00), Description, Par. No. [0002] Full text; Figs. 1 to 12 (Family: none) | 3,4,6-8,11, 12,14,15 |

☒ Further documents are listed in the continuation of Box C.　　☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 August, 2002 (21.08.02) | 03 September, 2002 (03.09.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.

PCT/JP02/04756

| | C (Continuation).   DOCUMENTS CONSIDERED TO BE RELEVANT | |
|---|---|---|
| Category* | Citation of document, with indication, where appropriate, of the relevant passages | Relevant to claim No. |
| A | JP 6-55145 A  (Satake Engineering Co., Ltd.), 01 March, 1994 (01.03.94), Full text; Figs. 1 to 6 (Family: none) | 1-18 |

Form PCT/ISA/210 (continuation of second sheet) (July 1998)